# EUROPEAN PATENT APPLICATION

(11) **EP 2 535 067 A1**
(43) Date of publication of application: **19.12.2012**
(21) Application number: 11742264.2
(22) Date of filing: 09.02.2011
(51) Int. Cl.: A61M 1/14

(54) **BLOOD PURIFICATION DEVICE AND PRIMING METHOD THEREFOR**

(30) Priority: 10.02.2010 JP 2010028182
(71) Applicant: Nikkiso Company Limited, Tokyo 150-6022 (JP)
(72) Inventor: SUZUKI, Tomohiro, Makinohara-shi Shizuoka 421-0496 (JP)
(74) Representative: von Hirschhausen, Helge
(86) International application number: PCT/JP2011/052754
(87) International publication number: WO 2011/099521

(57) **Abstract**

[Object] Disclosed is a blood purification apparatus that is capable of transitioning from priming to blood purification treatment while a fluid infusing line is still connected and is capable of improving workability during the transition from priming to blood purification treatment. Also disclosed is a priming method thereof.

[Solution] During priming, the other end of the fluid infusing line L3 is connected at the same location as the connected location of the fluid infusing line L3, which is determined in accordance with whether either pre-fluid infusion in which replenishment fluid is supplied to an arterial air trap chamber 5 or post-fluid infusion in which the replenishment fluid is supplied to a venous air trap chamber 6 will be performed in the blood purification treatment process, and the end of an arterial blood circuit 2 and the end of a venous blood circuit 3 are connected together to communicate fluid. In addition, a blood pump 4 is driven in normal rotation or in reverse rotation while the replenishment fluid is supplied from the fluid infusing line L3, thereby discharging the replenishment fluid from an overflow line 6a.

## Description

### Technical Field

The present invention relates to a blood purification apparatus which performs a blood purification treatment using a blood purifier connected to a blood circuit, and a priming method thereof.

### Background Art

Recently, in a dialysis apparatus as a blood purification apparatus, a technique has been suggested which performs priming, reinfusion, and fluid infusion (emergency fluid infusion) using a dialysate for being supplied to a dialyzer during dialysis treatment (particularly, an on-line HDF or an on-line HF). For example, Patent Document 1 discloses a dialysis apparatus which includes a fluid infusing line that has one end connected to a collection port formed in a predetermined part of a dialysate introduction line and the other end connected to a blood circuit (arterial blood circuit or venous blood circuit), and a fluid infusing pump disposed in the fluid infusing line. In order to perform the priming, the reinfusion or the fluid infusion (the emergency fluid infusion) using the dialysis apparatus, the dialysate in a dialysate introduction line is supplied to the blood circuit (the arterial blood circuit or the venous blood circuit) by driving the fluid infusing pump.

However, in a blood purifier (hereinafter, called an on-line HDF) which is applied to the blood dialysis filtration (HDF) and uses the dialysate as the fluid infusion, there is a need to perform the fluid infusion (including a pre-fluid infusion performing fluid infusion by the arterial blood circuit, and a post-fluid infusion performing fluid infusion by the venous blood circuit) of the dialysate to the patient's blood by the ultrafiltration corresponding to a filtration treatment as the HDF treatment. As an apparatus applied to the on-line HDF, as disclosed in PATENT DOCUMENT 2, a dialysis apparatus has been suggested which has a dialyzer, a blood circuit constituted by an arterial blood circuit and a venous blood circuit with a blood pump disposed thereon, a dialysate introduction line for introducing the dialysate into the dialyzer, a dialysate discharging line for discharging the dialysate from the dialyzer, and a fluid infusing line (a pre-fluid infusing line or a post-fluid infusing line) for supplying the dialysate of the dialysate introduction line to the blood circuit to perform the fluid infusion without going through the dialyzer.

### Citation List

### Patent Document

PATENT DOCUMENT 1: Japanese Laid-open Patent Publication No. 2004-313522
PATENT DOCUMENT 2: Japanese Laid-open Patent Publication No. 2001-112863

### Summary of Invention

### Technical Problem

However, in the blood purification apparatus of the related art mentioned above, the connection part of the fluid infusing line differs between priming and the blood purification treatment process. After priming is finished, a case of requiring the operation of connecting the fluid infusing line again is assumed. In that case, there has been a problem in that operability when changing from priming to blood purification treatment is degraded. However, such a problem can be similarly generated in the case of being applied to an off-line HDF (for example, the proximal end of the fluid supplement line is connected to an accommodating device that accommodates a replenishment fluid or the like) without being limited to the case of being applied to the on-line HDF as mentioned above.

The present invention has been made under such circumstances, and provides a blood purification apparatus that is able to transition from priming to blood purification treatment in the state of maintaining the connection state of the fluid infusing line, and is able to improve operability when being changed from priming to blood purification treatment, and a priming method thereof. Solution to Problem

According to the invention described in Claim 1, there is provided a blood purification apparatus that includes a blood purifier which includes a blood purification membrane and performs blood purification in the blood purification membrane; an arterial blood circuit, a proximal end of which is connected to the blood purifier and in which a blood pump is disposed in the middle thereof; a venous blood circuit, a proximal end of which is connected to the blood purifier; an arterial air trap chamber connected to the arterial blood circuit; a venous air trap chamber connected to the venous blood circuit; an overflow line which is extended from the top of the venous air trap chamber and can discharge liquid in the venous air trap chamber to the outside by causing the liquid to overflow; a dialysate introduction line which introduces a dialysate into the blood purifier; a dialysate discharge line which discharges the dialysate from the blood purifier; a fluid infusing line which can cause a replenishment fluid to flow in from one end thereof, and the other end of which can be connected to the arterial air trap chamber or the venous air trap chamber; and a fluid supplying device which is able to supply the replenishment fluid flowing in the fluid infusing line to the arterial blood circuit or the venous blood circuit via the arterial air trap chamber or the venous air trap chamber, wherein the other end of the fluid infusing line is connected to the same part as that of a blood purification treatment process during priming, and a tip of the arterial blood circuit is connected to a tip of the venous blood circuit in a communication state, and the blood purification apparatus further includes a control device capable of performing the control so as to discharge the replenishment fluid from the overflow line, by driving the blood pump in normal rotation or reverse rotation while supplying the replenishment fluid from the fluid infusing line.

According to the invention described in Claim 2, in the blood purification apparatus described in Claim 1, wherein one end of the fluid infusing line is connected to the dialysate introduction line, and the dialysate as the replenishment fluid is supplied to the arterial blood circuit or the venous blood circuit.

According to the invention described in Claim 3, in the blood purification apparatus described in Claim 1 or 2, wherein the fluid supplying device includes a fluid infusing pump disposed in the fluid infusing line.

According to the invention described in Claim 4, in the blood purification apparatus described in any one of Claims 1 to 3, wherein the proximal end of the fluid infusing line is connected to the arterial air trap chamber and a pre-fluid infusion is performed in the blood purification treatment process characterized in that during priming, the control device drives the blood pump in reverse rotation and controls a drive speed of the blood pump so as to be equal to or less than a supply speed of the replenishment fluid using the fluid supplying device.

According to the invention described in Claim 5, in the blood purification apparatus described in any one of Claims 1 to 3, wherein the proximal end of the fluid infusing line is connected to the venous air trap chamber and a post-fluid infusion is performed in the blood purification treatment process, characterized in that during priming, the control device drives the blood pump in normal rotation and controls a drive speed of the blood pump so as to be equal to or less than a supply speed of the replenishment fluid using the fluid supplying device.

According to the invention described in Claim 6, in the blood purification apparatus described in Claim 5, wherein an air bubble detection device is disposed at a tip side of the venous blood circuit, and during priming, the control device sequentially performs a first circulation process of driving the blood pump in reverse rotation at a predetermined speed, and a second circulation process of driving the blood pump at a speed lower than the predetermined speed with the detection of air bubbles by the air bubble detection device in the first circulation process as a condition.

According to the invention described in Claim 7, there is provided a priming method of a blood purification apparatus that includes a blood purifier which includes a blood purification membrane and performs blood purification in the blood purification membrane; an arterial blood circuit, a proximal end of which is connected to the blood purifier and in which a blood pump is disposed in the middle thereof; a venous blood circuit, a proximal end of which is connected to the blood purifier; an arterial air trap chamber connected to the arterial blood circuit; a venous air trap chamber connected to the venous blood circuit; an overflow line which is extended from the top of the venous air trap chamber and can discharge liquid in the venous air trap chamber to the outside by causing the liquid to overflow; a dialysate introduction line which introduces a dialysate into the blood purifier; a dialysate discharge line which discharges the dialysate from the blood purifier; a fluid infusing line which can cause a replenishment fluid to flow in from one end thereof, and the other end of which can be connected to the arterial air trap chamber or the venous air trap chamber; and a fluid supplying device which is able to supply the replenishment fluid flowing in the fluid infusing line to the arterial blood circuit or the venous blood circuit via the arterial air trap chamber or the venous air trap chamber, wherein the other end of the fluid infusing line is connected to the same site as that of a blood purification treatment process during priming, and a tip of the arterial blood circuit is connected to a tip of the venous blood circuit in a communication state, and the replenishment fluid is discharged from the overflow line by driving the blood pump in normal rotation or reverse rotation while supplying the replenishment fluid from the fluid infusing line.

According to the invention described in Claim 8, in the priming method of the blood purification apparatus described in Claim 7, wherein one end of the fluid infusing line is connected to the dialysate introduction line, and the dialysate as the replenishment fluid is supplied to the arterial blood circuit or the venous blood circuit.

According to the invention described in Claim 9, in the priming method of the blood purification apparatus described in Claim 7 or 8, wherein the fluid supplying device includes a fluid infusing pump disposed in the fluid infusing line.

According to the invention described in Claim 10, in the priming method of the blood purification apparatus described in any one of Claims 7 to 9, wherein the proximal end of the fluid infusing line is connected to the arterial air trap chamber and a pre-fluid infusion is performed in the blood purification treatment process, characterized in that during priming, the blood pump is driven in reverse rotation and a drive speed of the blood pump is set to be equal to or less than a supply speed of the replenishment fluid using the fluid supplying device.

According to the invention described in Claim 11, in the priming method of the blood purification apparatus described in any one of Claims 7 to 9, wherein the proximal end of the fluid infusing line is connected to the venous air trap chamber and a post-fluid infusion is performed in the blood purification treatment process, characterized in that during priming, the blood pump is driven in normal rotation and a drive speed of the blood pump is set to be equal to or less than a supply speed of the replenishment fluid using the fluid supplying device.

According to the invention described in Claim 12, in the priming method of the blood purification apparatus described in Claim 11, wherein an air bubble detection device is disposed at a tip side of the venous blood circuit, and during priming, the control device sequentially performs a first circulation process of driving the blood pump in reverse rotation at a predetermined speed, and a second circulation process of driving the blood pump at a speed lower than the predetermined speed as a condition that the air bubble detection device detects air bubbles in the first circulation process.

### Advantageous Effects of Invention

According to the present invention, during priming, the other end of the fluid infusing line is connected to the same site as that of the blood purification treatment process, the tip of the arterial blood circuit is connected to the tip of the venous blood circuit in the communication state, and the replenishment fluid is discharged from the overflow line by driving the blood pump in normal rotation or reverse rotation while supplying the replenishment fluid from the fluid infusing line. Thus, the operation can be shifted from priming to blood purification treatment in a state maintaining the connection state of the fluid infusing line, whereby it is possible to improve operability when shifting from priming to blood purification treatment. Brief Description of Drawings

[Fig. 1] Fig. 1 is a schematic diagram that shows a dialysis apparatus according to a first embodiment of the present invention.
[Fig. 2] Fig. 2 is a schematic diagram that shows a state when performing priming (air purging) in the dialysis apparatus.
[Fig. 3] Fig. 3 is a schematic diagram that shows a state when performing priming (cleaning) in the dialysis apparatus.
[Fig. 4] Fig. 4 is a schematic diagram that shows a dialysis apparatus according to a second embodiment of the present invention.
[Fig. 5] Fig. 5 is a schematic diagram that shows a state when performing priming (air purging) in the dialysis apparatus.
[Fig. 6] Fig. 6 is a schematic diagram that shows a state when performing priming (a first circulation process) in the dialysis apparatus.
[Fig. 7] Fig. 7 is a schematic diagram that shows a state when performing priming (a second circulation process) in the dialysis apparatus.

### Description of Embodiments

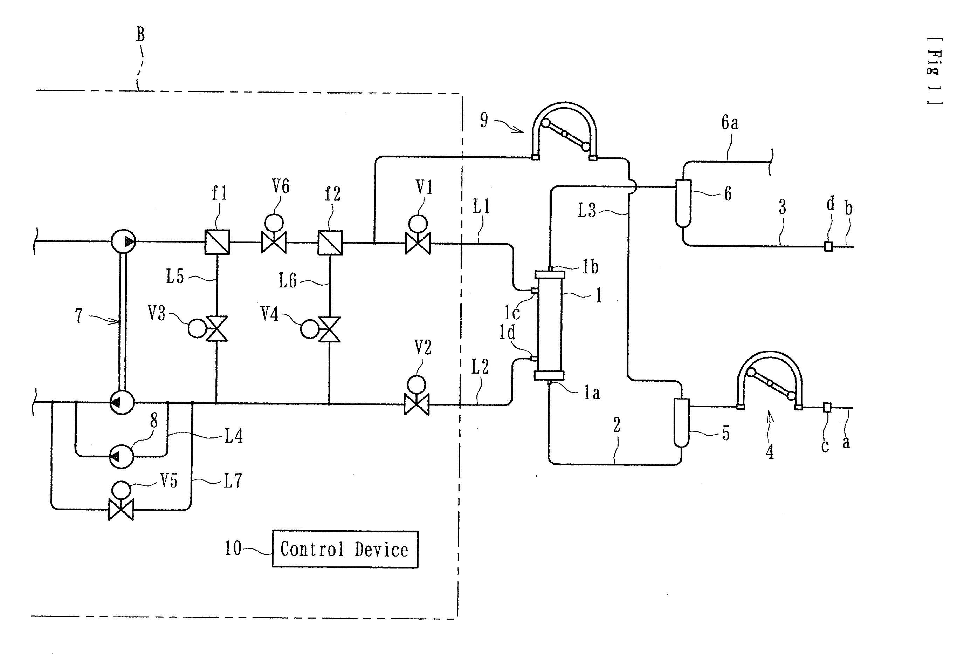

Hereinafter, an embodiment of the present invention will be specifically described with reference to the drawings.
A blood purification apparatus according to the present embodiment is applied to a blood dialysis apparatus which performs a pre-fluid infusion that supplies a dialysate as a replenishment fluid to an arterial blood circuit 2 in the blood purification treatment process. As shown in Fig. 1, the blood purification apparatus mainly includes a blood circuit in which the arterial blood circuit 2 and a venous blood circuit 3 are connected to a dialyzer 1 as a blood purifier, a dialysis apparatus main body B having a dialysate introduction line L1 and a dialysate discharging line L2, a fluid infusing line L3, a fluid infusing pump 9 as a fluid supplying device, and a control device 10.

The dialyzer 1 includes a blood purification membrane (not shown) (although the membrane is a hollow fiber type blood dialysis filtration membrane in the present embodiment, the membrane includes a flat membrane type, blood dialysis membrane, and a blood filtration membrane). The dialyzer 1 is formed with a blood introduction port 1a for introducing the blood and a blood discharge port 1b for discharging the introduced blood, is formed with a dialysate introduction port 1c for introducing the dialysate and a dialysate discharge port 1d for discharging the introduced dialysate, and purifies the blood by bringing the dialysate into contact with the blood introduced from the blood introduction portion 1a via a hollow fiber. Furthermore, the dialyzer 1 is mounted so that the blood introduction port 1a faces downward.

The arterial blood circuit 2 is mainly constituted by a flexible tube, and one end thereof is connected to a blood introduction portion 1a of the dialyzer 1 to guide the blood collected from a patient's blood vessel into the hollow fiber of the dialyzer 1. In the other end of the arterial blood circuit 2, a connector c capable of attaching an arterial puncture needle a is formed, an arterial air trap chamber 5 is connected to the middle thereof, and a blood pump 4 is disposed. Since the arterial air trap chamber 5 is mounted in the same direction as that when performing the dialysis treatment, it is possible to eliminate the work of inverting the arterial air trap chamber 5 before the dialysis treatment is started. Furthermore, the blood pump 4 is a peristaltic type pump (having a configuration that squeezes the flexible tube during normal rotation to cause the blood from the arterial puncture needle a to flow in a direction of the blood introduction port 1a of the dialyzer 1) .

The venous blood circuit 3 is mainly constituted by the flexible tube as in the arterial blood circuit 2, and one end thereof is connected to a blood introduction port 1b of the dialyzer 1 to introduce the blood passing through the hollow fiber. In the other end of the venous blood circuit 3, a connecter d capable of attaching a venous puncture needle b is formed, and a venous air trap chamber 6 is connected to the middle thereof. Since the venous air trap chamber 6 is mounted in the same direction as that when performing the dialysis treatment, it is possible to eliminate a task of inverting the venous air trap chamber 6 before the dialysis treatment is started. An overflow line 6a capable of discharging liquid in the venous air trap chamber by causing the liquid to overflow is extended from the top of the venous air trap chamber 6. Furthermore, the patient's blood collected by the arterial puncture needle a reaches the dialyzer 1 via the arterial blood circuit 2, flows through the venous blood circuit 3 after the blood purification is performed, and returns back to the body of the patient via the venous puncture needle b, whereby the extracorporeal circulation is performed.

The dialysate introduction line L1 and the dialysate discharge line L2 are connected to the dialysate introduction port 1c and the dialysate discharge port 1d of the dialyzer 1, respectively, and the dialysate introduced to the dialyzer 1 via the dialysate introduction line L1 can be discharged from the dialysate discharge line L2 through the outside of the hollow fiber membrane. An electromagnetic valve V1 and an electromagnetic valve V2 are connected to the middle of the dialysate introduction line L1 and the dialysate discharge line L2, respectively.

Furthermore, a duplex pump 7, which supplies the dialyzer 1 with the dialysate prepared to a predetermined concentration and discharges the dialysate from the dialyzer 1, is connected to the dialysate introduction line L1 and the dialysate discharge line L2. Furthermore, bypass lines L5 and L6, which cause the dialysate introduction line L1 and the dialysate discharge line L2 to communicate with each other, are disposed in the dialysis apparatus main body B, and electromagnetic valves V3 and V4 are disposed in the middle of the bypass lines L5 and L6, respectively. Furthermore, in the drawings, reference numerals f1 and f2 indicate filtration filters disposed in the dialysate introduction line L1, and an electromagnetic valve V6 is disposed between the filtration filters f1 and f2.

Meanwhile, the dialysate discharge line L2 is formed with bypass lines L4 and L7 that bypass the duplex pump 7, an ultrafiltration pump 8 for removing the water content from the patient's blood flowing in the dialyzer 1 is disposed in the bypass line L4, and the electromagnetic valve V5 capable of opening or closing the flow route is disposed in the bypass line L7.

One end of the fluid infusing line L3 is connected to a collection port (not shown) formed in a predetermined location of the dialysate introduction line L1, the dialysate (replenishment fluid) can flow in from the one end, and the other end thereof is constituted by a flow route (for example, a flexible tube or the like) connected to the top of the arterial air trap chamber 5. The collection port is constituted by a port formed in the dialysis apparatus main body B, and the dialysate introduction line L1 and the arterial air trap chamber 5 can be caused to communicate with each other, by connecting one end of the fluid infusing line L3 to the collection port.

The fluid infusing pump 9 as the fluid supplying device is disposed in the fluid infusing line L3 and is able to supply the dialysate (replenishment fluid) flowing in the fluid infusing line L3 to the arterial blood circuit 2 via the arterial air trap chamber 5. As a result, one end and the other end of the fluid infusing line L3 are connected to the dialysate introduction line L1 and the arterial air trap chamber 5, respectively, and by driving (normal rotation) the fluid infusing pump 9, it is possible to perform the pre-fluid infusion (a fluid infusion form that supplies the dialysate as the replenishment fluid to the arterial blood circuit 2) in the blood purification treatment process. Furthermore, as in the blood pump 4, the fluid infusing pump 9 is a peristaltic type pump (having a configuration that can squeeze the tube constituting the fluid infusing line L3 when being driven to cause the dialysate to flow).

However, the fluid infusing line L3 is disposed with a clamp device (not shown) capable of opening and closing the flow route thereof. After the fluid infusing line L3 is connected to the collection port by a worker, the clamp device is in the closed state until the dialysate is caused to circulate, and the flow route is closed. Moreover, if necessary (during priming, reinfusion, fluid infusion or the like), the clamp device is in the open state by a worker, and the dialysate introduction line L1 communicates with the blood circuit (arterial blood circuit 2).

The control device 10 is formed of, for example, a microcomputer or the like that can control the opening and the closing of various electromagnetic valves V1 to V6 disposed in the dialysis apparatus and an actuator of the blood pump 4, the fluid infusing pump 9 or the like. Particularly, in the present embodiment, it is possible to perform the control so as to drive the blood pump 4 in reverse rotation while supplying the dialysate (replenishment fluid) from the fluid infusing line L3 and discharge the dialysate (replenishment fluid) from the overflow line 6a, by driving the fluid infusing pump 9 during priming.

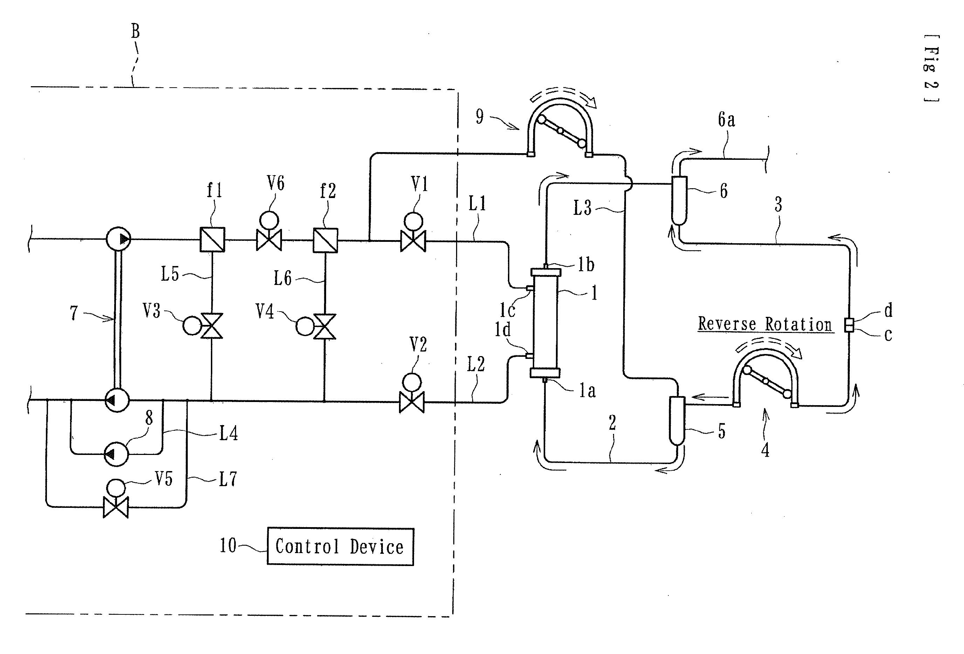

More specifically, during priming (particularly, during an air purging process in the blood circuit and in the blood flow route of the dialyzer 1), as shown in Fig. 2, the other end of the fluid infusing line L3 is connected to the same site (because the pre-fluid infusion is performed in the blood purification treatment process in the present embodiment, the top of the arterial air trap chamber 5) as the connection site of the fluid infusing line L3 which is determined depending on whether any one of the pre-fluid infusion for supplying the dialysate (replenishment fluid) to the arterial air trap chamber 5 and the post-fluid infusion for supplying the dialysate (replenishment fluid) to the venous air trap chamber 6 is performed in the blood purification treatment process, and the tip (connector c) of the arterial blood circuit 2 is connected to the tip (connector d) of the venous blood circuit 3 in the communication state.

In such a state, the blood pump 4 is driven in reverse rotation while supplying the dialysate (replenishment fluid) from the fluid infusing line L3 by driving the fluid infusing pump 9 through the control using the control device 10, and the dialysate (replenishment fluid) is discharged from the overflow line 6a. At this time, the blood pump 4 is driven in reverse rotation by the control device 10, and the drive speed (flow speed) of the blood pump 4 is controlled so as to be equal to or less than the supply speed (flow speed) of the dialysate (replenishment fluid) using the fluid infusing pump 9 (fluid supplying device).

Furthermore, a part of the dialysate (replenishment fluid) supplied to the arterial air trap chamber 5 by the fluid infusing line L3 is divided into the flow toward the connection section (the connection sections of the connectors c and d) between the tip of the arterial blood circuit 2 and the tip of the venous blood circuit 3 and the flow toward the dialyzer 1, the respective flows are joined to each other in the venous air trap chamber 6, and are discharged from the overflow line 6a to the outside. In the process of the flow of the dialysate (replenishment fluid), air in the blood circuit and the blood flow route of the dialyzer 1 is discharged to the outside via the overflow line 6a.

According to the present embodiment, during priming, the other end of the fluid infusing line L3 is connected to the same site as the connection site of the fluid infusing line L3 which is determined depending on whether any one of the pre-fluid infusion for supplying the dialysate (replenishment fluid) to the arterial air trap chamber 5 and the post-fluid infusion for supplying the dialysate (replenishment fluid) to the venous air trap chamber 6 is performed in the blood purification treatment process, and the tip of the arterial blood circuit 2 is connected to the tip of the venous blood circuit 3 in the communication state. Furthermore, the blood pump 4 is driven in reverse rotation while supplying the dialysate (replenishment fluid) from the fluid infusing line L3, thereby discharging the dialysate (replenishment fluid) from the overflow line 6a. Thus, the operation can be shifted from priming to blood purification treatment in a state maintaining the connection state of the fluid infusing line L3, and it is possible to improve operability when being shifted from priming to blood purification treatment.

Furthermore, according to the present embodiment, the control device 10 drives the blood pump 4 in reverse rotation and controls the drive speed (flow speed) of the blood pump 4 so as to be equal to or less than the supply speed (flow speed) of the dialysate (replenishment fluid) using the fluid infusing pump 9 (fluid supplying device). Thus, priming can be performed by causing the dialysate (replenishment fluid) to flow in any of the arterial blood circuit 2 and the venous blood circuit 3, whereby more reliable and smoother priming (air purging) can be performed.

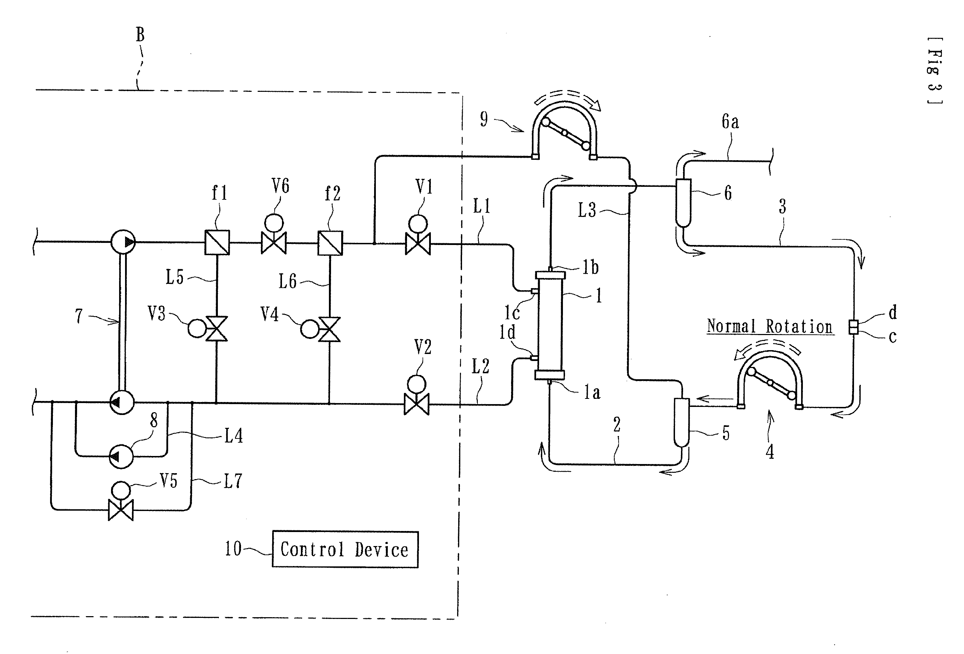

Furthermore, in the present embodiment, after determining that air purging is finished after a predetermined time elapses or in an amount of rotation of the blood pump 4 or the fluid infusing pump 9, as shown in Fig. 3, the control using the control device 10 is performed so as to drive the blood pump 4 in normal rotation while maintaining the driving of the fluid infusing pump 9. As a result, the dialysate (replenishment fluid) is circulated in the blood circuit and the blood flow route of the dialyzer 1 in one direction, and the dialysate (replenishment fluid) can be discharged from the overflow line 6a to the outside, whereby the cleaning process can be performed after air purging in the priming process.

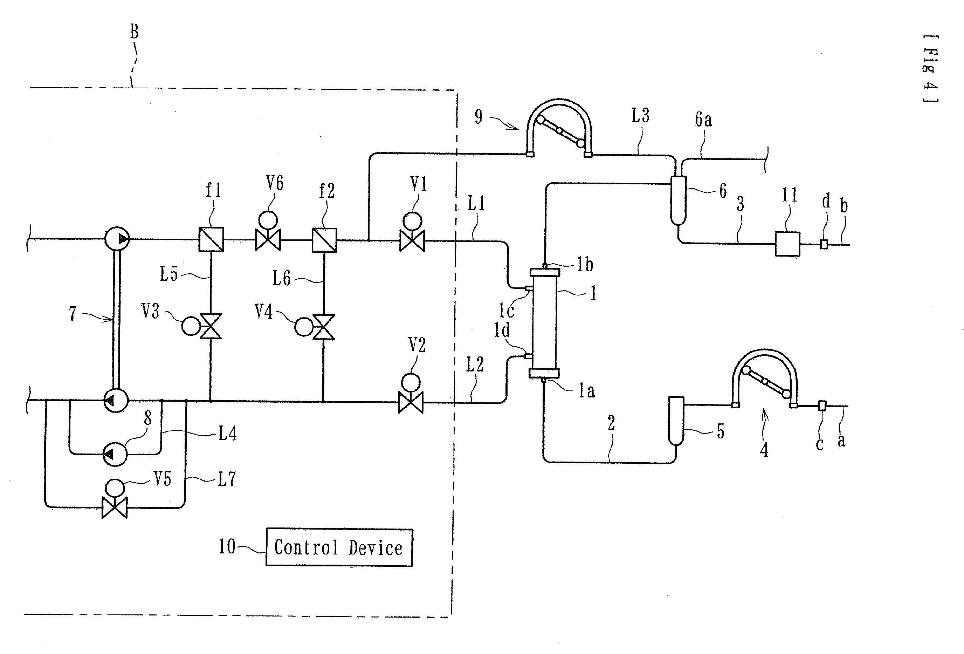

Next, a second embodiment of the present invention will be described.
A blood purification apparatus according to the present embodiment is applied to a blood dialysis apparatus which performs a post-fluid infusion that supplies the dialysate as a replenishment fluid to a venous blood circuit 3 in the blood purification treatment process. As shown in Fig. 4, the blood purification apparatus mainly includes a blood circuit in which the arterial blood circuit 2 and a venous blood circuit 3 are connected to a dialyzer 1 as a blood purifier, a dialysis apparatus main body B having a dialysate introduction line L1 and a dialysate discharging line L2, a fluid infusing line L3, a fluid infusing pump 9 as a fluid supplying device, a control device 10, and an air bubble detection device 11.

The air bubble detection device 11 is constituted by a sensor which is disposed on the tip side (near the connector d) of the venous blood circuit 3 to detect the air bubbles in the venous blood circuit 3, and is constituted so as to detect the presence or the absence of the air bubbles in the blood circulating extracorporeally. As a result, in the blood purification treatment process, when the air bubbles are detected by the air bubble detection device 11, the extracorporeal circulation is stopped and stability can be improved.

Herein, in the present embodiment, one end of the fluid infusing line L3 is connected to a collection port (not shown) formed in a predetermined location of the dialysate introduction line L1, the dialysate (replenishment fluid) can flow in from the one end, and the other end thereof is constituted by a flow route (for example, a flexible tube or the like) connected to the top of the venous air trap chamber 6. The collection port is constituted by a port formed in the dialysis apparatus main body B, and the dialysate introduction line L1 and the venous air trap chamber 6 can be caused to communicate with each other, by connecting one end of the fluid infusing line L3 to the collection port.

The fluid infusing pump 9 as the fluid supplying device is disposed in the fluid infusing line L3 and is able to supply the dialysate (replenishment fluid) flowing in the fluid infusing line L3 to the venous blood circuit 3 via the venous air trap chamber 6. As a result, one end and the other end of the fluid infusing line L3 are connected to the dialysate introduction line L1 and the venous air trap chamber 6, respectively, and by driving (normal rotation) the fluid infusing pump 9, it is possible to perform the post-fluid infusion (a fluid infusion form that supplies the dialysate as the replenishment fluid to the venous blood circuit 3) in the blood purification treatment process. Furthermore, as in the first embodiment, the fluid infusing pump 9 is a peristaltic type pump (having a configuration that can squeeze the tube constituting the fluid infusing line L3 when being driven to cause the dialysate to flow).

However, the fluid infusing line L3 is disposed with a clamp device (not shown) capable of opening and closing the flow route thereof, similar to the first embodiment. After the fluid infusing line L3 is connected to the collection port by a worker, the clamp device is in the closed state until the dialysate is caused to circulate, and the flow route is closed. Moreover, if necessary (during priming, reinfusion, fluid infusion or the like), the clamp device is in the open state by a worker, and the dialysate introduction line L1 communicates with the blood circuit (venous blood circuit 3).

As in the first embodiment, the control device 10 is formed of, for example, a microcomputer or the like that can control the opening and the closing of various electromagnetic valves V1 to V6 disposed in the dialysis apparatus and an actuator of the blood pump 4, the fluid infusing pump 9 or the like. Particularly, in the present embodiment, it is possible to perform the control so as to drive the blood pump 4 in normal rotation while supplying the dialysate (replenishment fluid) from the fluid infusing line L3 and discharge the dialysate (replenishment fluid) from the overflow line 6a, by driving the fluid infusing pump 9 during priming.

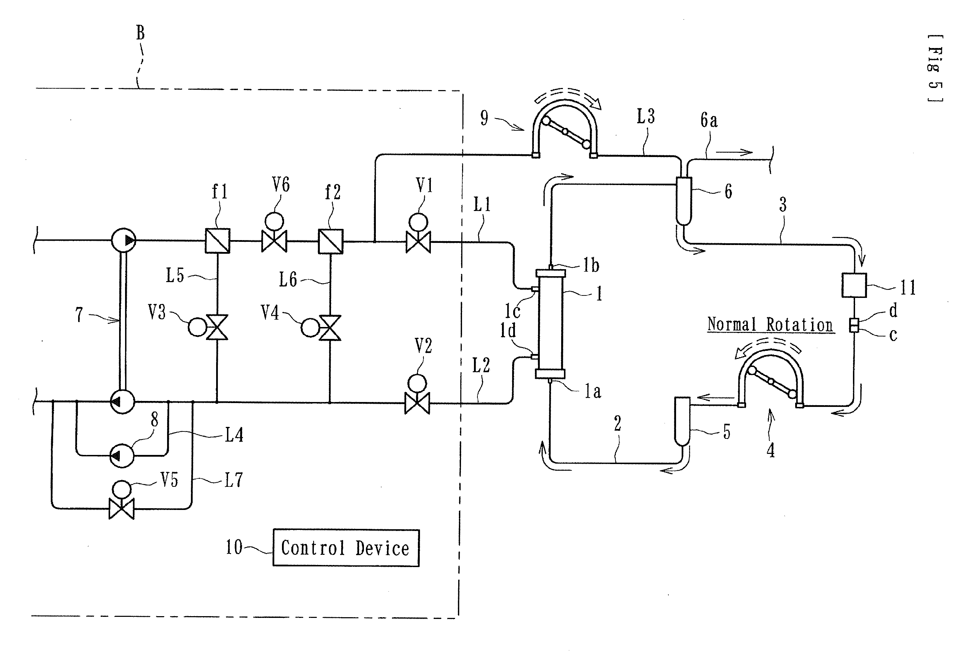

More specifically, during priming (particularly, during an air purging process in the blood circuit and in the blood flow route of the dialyzer 1), as shown in Fig. 5, the other end of the fluid infusing line L3 is connected to the same site (because the post-fluid infusion is performed in the blood purification treatment process in the present embodiment, the top of the venous air trap chamber 6) as the connection site of the fluid infusing line L3 which is determined depending on whether any one of the pre-fluid infusion for supplying the dialysate (replenishment fluid) to the arterial air trap chamber 5 and the post-fluid infusion for supplying the dialysate (replenishment fluid) to the venous air trap chamber 6 is performed in the blood purification treatment process, and the tip (connector c) of the arterial blood circuit 2 is connected to the tip (connector d) of the venous blood circuit 3 in the communication state.

In such a state, the blood pump 4 is driven in normal rotation while supplying the dialysate (replenishment fluid) from the fluid infusing line L3 by driving the fluid infusing pump 9 through the control using the control device 10, and the dialysate (replenishment fluid) is discharged from the overflow line 6a. At this time, the blood pump 4 is driven in normal rotation by the control device 10, and the drive speed (flow speed) of the blood pump 4 is controlled so as to be equal to or less than the supply speed (flow speed) of the dialysate (replenishment fluid) using the fluid infusing pump 9 (fluid supplying device). Furthermore, by driving only the fluid infusing pump 9 in advance to inject the dialysate (replenishment fluid) to the venous air trap chamber 6, air to be sent to the dialyzer 1 can be reduced, whereby the more efficient priming is possible.

Furthermore, a part of the dialysate (replenishment fluid) supplied to the venous air trap chamber 6 by the fluid infusing line L3 becomes the flow toward the connection section (the connection sections of the connectors c and d) between the tip of the arterial blood circuit 2 and the tip of the venous blood circuit 3, and the other part thereof becomes the flow discharged from the overflow line 6a. However, in a case where the drive speed (flow speed) of the blood pump 4 is equal to the supply speed (flow speed) of the dialysate (replenishment fluid) using the fluid infusing pump 9 (fluid supplying device), the other part can also become the flow toward the connection section side between the tip of the arterial blood circuit 2 and the tip of the venous blood circuit 3. In the process of the flow of the dialysate (replenishment fluid), air in the blood circuit and the blood flow route of the dialyzer 1 is discharged to the outside via the overflow line 6a.

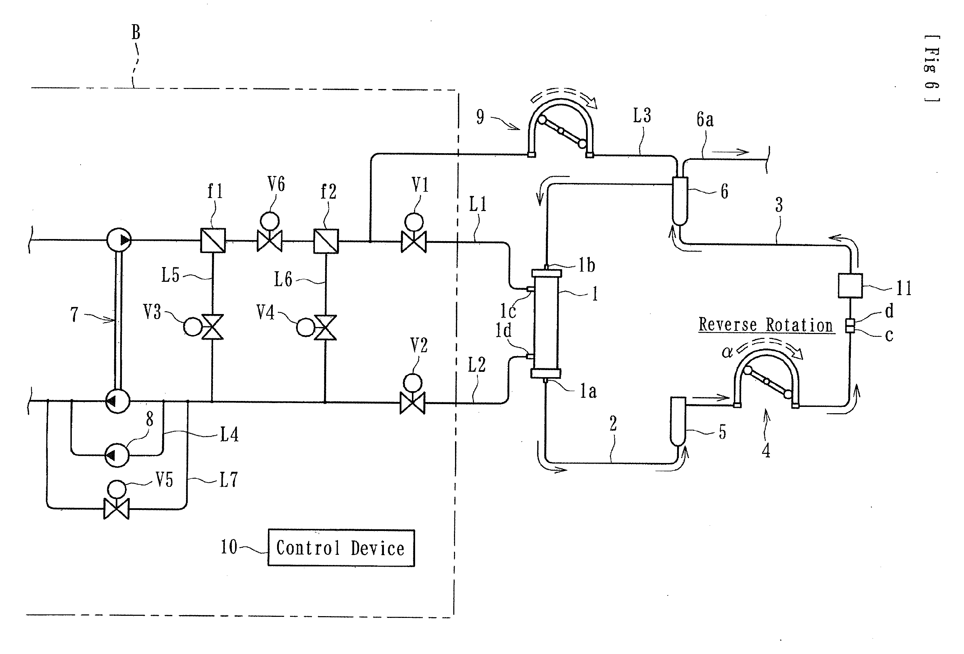

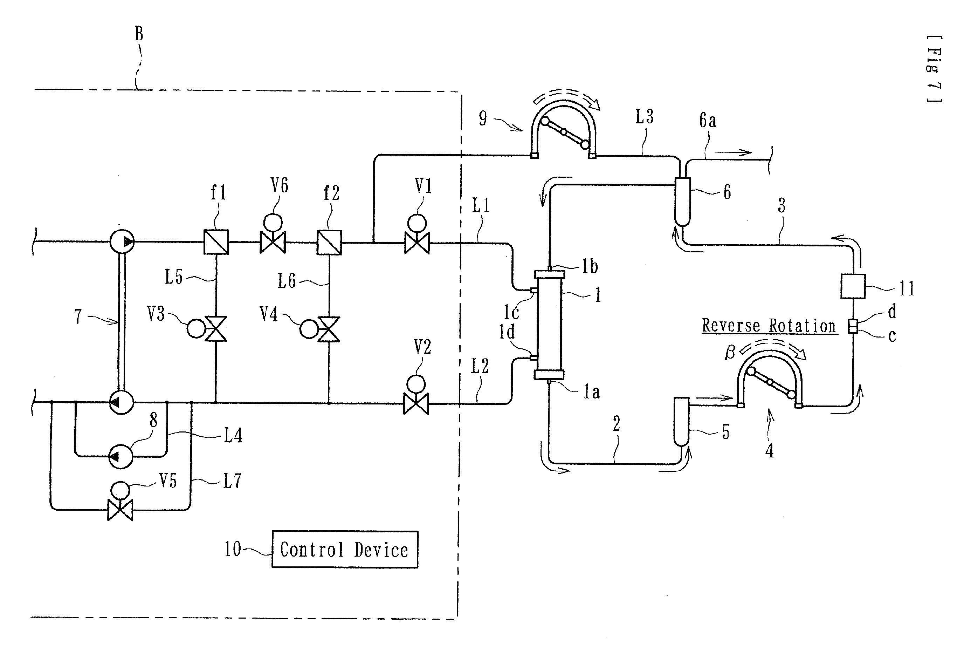

After performing the process as mentioned above, the control device 10 performs a first circulation process of driving the blood pump 4 in reverse rotation at a high speed (for example, a flow speed α) as shown in Fig. 6, and performs a second circulation process of driving the blood pump 4 in reverse rotation at low speeds (for example, a flow speed β slower than the flow speed α) as the condition that the air bubble detection device 11 detects the air bubbles in the first circulation process, as shown in Fig. 7. As a result, it is possible to reliably capture the air bubbles trapped in the arterial air trap chamber 5 by the venous air trap chamber 6 and discharge the air bubbles to the outside via the overflow line 6a, whereby it is possible to reliably prevent the air bubbles from reaching the dialyzer 1. Furthermore, priming can be completed at an early stage. Herein, by always driving the blood pump 4 in reverse rotation at the flow speed α to perform priming, the air bubble detection device 11 can be eliminated. However, in that case, since it is required for a long time, it is preferable to perform the air bubble detection using the air bubble detection device 11 as mentioned above.

In the first circulation process and the second circulation process according to the present embodiment, the fluid infusing pump 9 may be driven or may be stopped, and the flow speeds α and β can be set to an arbitrary drive speed regardless of the driving speed of the fluid infusion speed. Moreover, as a condition that the air bubbles are not detected for a predetermined time by the air bubble detection device 11, priming (air purging) is finished. Furthermore, after performing air purging, as in the first embodiment (see Fig. 3), the control of the control device 10 is performed so as to drive the blood pump 4 in normal rotation while maintaining the driving of the fluid infusing pump 9. As a result, the dialysate (replenishment fluid) is circulated in one direction in the blood circuit and the blood flow route of the dialyzer 1, and the dialysate (replenishment fluid) can be discharged from the overflow line 6a to the outside, whereby the cleaning process can be performed after air purging in the priming process.

According to the present embodiment, during priming, the other end of the fluid infusing line L3 is connected to the same site as the connection site of the fluid infusing line L3 which is determined depending on whether any one of the pre-fluid infusion for supplying the dialysate (replenishment fluid) to the arterial air trap chamber 5 or the post-fluid infusion for supplying the dialysate (replenishment fluid) to the venous air trap chamber 6 is performed in the blood purification treatment process, and the tip of the arterial blood circuit 2 is connected to the tip of the venous blood circuit 3 in the communication state. Furthermore, the blood pump 4 is driven in reverse rotation while supplying the dialysate (replenishment fluid) from the fluid infusing line L3, thereby discharging the dialysate (replenishment fluid) from the overflow line 6a. Thus, the operation can be shifted from priming to blood purification treatment in a state maintaining the connection state of the fluid infusing line L3, and it is possible to improve operability when being shifted from priming to blood purification treatment.

Furthermore, according to the present embodiment, the control device 10 drives the blood pump 4 in normal rotation and controls the drive speed (flow speed) of the blood pump 4 so as to be equal to or less than the supply speed (flow speed) of the dialysate (replenishment fluid) using the fluid infusing pump 9 (fluid supplying device). Thus, priming can be performed by causing the dialysate (replenishment fluid) to flow in any of the arterial blood circuit 2 and the venous blood circuit 3, whereby more reliable and smoother priming (air purging) can be performed.

Furthermore, in the present embodiment, since the control device 10 sequentially performs the first circulation process of driving the blood pump 4 in reverse rotation at a high speed, and the second circulation process of driving the blood pump 4 in reverse rotation at low speeds as the condition that the air bubble detection device 11 detects the air bubbles in the first circulation process, it is possible to smoothly perform the premature termination of priming and the removal of the air bubbles in the arterial air trap chamber 5. Thus, since there is no need to invert the arterial air trap chamber 5, it is possible to further improve operability during priming and when being shifted from priming to blood purification treatment.

Although the embodiments have been described as above, the present invention is not limited thereto. The present invention is not limited to, for example, the application to the on-line HDF, but may be applied to the off-line HDF (for example, the proximal end of the fluid infusing line is connected to the accommodating device for accommodating the replenishment fluid (a so-called fluid infusion which is not limited to the dialysate) or the like). Furthermore, in the present embodiments, although the fluid infusing pump 9 as the fluid supplying device is disposed in the fluid infusing line L3, for example, another pump (a duplex pump 7, a cascade pump or the like in the present embodiment) may be used as the fluid supplying device. Furthermore, in the second embodiment, although the air bubble detection device 11 is disposed at the tip side of the venous blood circuit 3, the air bubble detection device may not be disposed. Alternately, in the first embodiment, the air bubble detection device 11 may be disposed at the tip side of the venous blood circuit 3.

### Industrial Applicability

As long as there is provided a blood purification apparatus and a priming method thereof in which, during priming, the other end of the fluid infusing line is connected to the same site as the connection site of the fluid infusing line which is determined depending on whether any one of the pre-fluid infusion for supplying the replenishment fluid to the arterial air trap chamber or the post-fluid infusion for supplying the replenishment fluid to the venous air trap chamber is performed in the blood purification treatment process, the tip of the arterial blood circuit is connected to the tip of the venous blood circuit in the communication state, and the blood pump is driven in reverse rotation while supplying the replenishment fluid from the fluid infusing line, thereby discharging the replenishment fluid from the overflow line, the present invention can be applied to an apparatus with other functions added thereto.

### Reference Signs List

- 1: dialyzer (blood purifier)
- 2: arterial blood circuit
- 3: venous blood circuit
- 4: blood pump
- 5: arterial air trap chamber
- 6: venous air trap chamber
- 6a: overflow line
- 7: duplex pump
- 8: ultrafiltration pump
- 9: fluid infusing pump (fluid supplying device)
- 10: control device
- 11: air bubble detection device
- L1: dialysate introduction line
- L2: dialysate discharging line
- L3: fluid infusing line

## Claims

1. A blood purification apparatus comprising:
a blood purifier which includes a blood purification membrane and performs blood purification in the blood purification membrane;
an arterial blood circuit, a proximal end of which is connected to the blood purifier and in which a blood pump is disposed in the middle thereof;
a venous blood circuit, a proximal end of which is connected to the blood purifier;
an arterial air trap chamber connected to the arterial blood circuit;
a venous air trap chamber connected to the venous blood circuit;
an overflow line which is extended from the top of the venous air trap chamber and can discharge liquid in the venous air trap chamber to the outside by causing the liquid to overflow;
a dialysate introduction line which introduces a dialysate into the blood purifier;
a dialysate discharge line which discharges the dialysate from the blood purifier;
a fluid infusing line which can cause a replenishment fluid to flow in from one end thereof, and the other end of which can be connected to the arterial air trap chamber or the venous air trap chamber; and
a fluid supplying device which is able to supply the replenishment fluid flowing in the fluid infusing line to the arterial blood circuit or the venous blood circuit via the arterial air trap chamber or the venous air trap chamber,
wherein the other end of the fluid infusing line is connected to the same site as that of a blood purification treatment process during priming, and a tip of the arterial blood circuit is connected to a tip of the venous blood circuit in a communication state, and the blood purification apparatus further includes a control device capable of performing the control so as to discharge the replenishment fluid from the overflow line, by driving the blood pump in normal rotation or reverse rotation while supplying the replenishment fluid from the fluid infusing line.

2. The blood purification apparatus according to Claim 1,
wherein one end of the fluid infusing line is connected to the dialysate introduction line, and the dialysate as the replenishment fluid is supplied to the arterial blood circuit or the venous blood circuit.

3. The blood purification apparatus according to Claim 1 or 2,
wherein the fluid supplying device includes a fluid infusing pump disposed in the fluid infusing line.

4. The blood purification apparatus according to any one of Claims 1 to 3,
wherein a proximal end of the fluid infusing line is connected to the arterial air trap chamber and a pre-fluid infusion is performed in the blood purification treatment process,
**characterized in that** during priming, the control device drives the blood pump in reverse rotation and controls a drive speed of the blood pump so as to be equal to or less than a supply speed of the replenishment fluid using the fluid supplying device.

5. The blood purification apparatus according to any one of Claims 1 to 3,
wherein the proximal end of the fluid infusing line is connected to the venous air trap chamber and a post-fluid infusion is performed in the blood purification treatment process,
**characterized in that** during priming, the control device drives the blood pump in normal rotation and controls a drive speed of the blood pump so as to be equal to or less than a supply speed of the replenishment fluid using the fluid supplying device.

6. The blood purification apparatus according to Claim 5,
wherein an air bubble detection device is disposed at a tip side of the venous blood circuit, and during priming, the control device sequentially performs a first circulation process of driving the blood pump in reverse rotation at a predetermined speed, and a second circulation process of driving the blood pump at a speed lower than the predetermined speed as a condition that the air bubble detection device detects air bubbles in the first circulation process.

7. A priming method of a blood purification apparatus comprising:
a blood purifier which includes a blood purification membrane and performs blood purification in the blood purification membrane;
an arterial blood circuit, a proximal end of which is connected to the blood purifier and in which a blood pump is disposed in the middle thereof;
a venous blood circuit, a proximal end of which is connected to the blood purifier;
an arterial air trap chamber connected to the arterial blood circuit; a venous air trap chamber connected to the venous blood circuit;
an overflow line which is extended from the top of the venous air trap chamber and can discharge liquid in the venous air trap chamber to the outside by causing the liquid to overflow;
a dialysate introduction line which introduces a dialysate into the blood purifier;
a dialysate discharge line which discharges the dialysate from the blood purifier;
a fluid infusing line which can cause a replenishment fluid to flow in from one end thereof, and the other end of which can be connected to the arterial air trap chamber or the venous air trap chamber; and
a fluid supplying device which is able to supply the replenishment fluid flowing in the fluid infusing line to the arterial blood circuit or the venous blood circuit via the arterial air trap chamber or the venous air trap chamber,
wherein the other end of the fluid infusing line is connected to the same site as that of a blood purification treatment process during priming, and a tip of the arterial blood circuit is connected to a tip of the venous blood circuit in a communication state, and the replenishment fluid is discharged from the overflow line by driving the blood pump in normal rotation or reverse rotation while supplying the replenishment fluid from the fluid infusing line.

8. The priming method of the blood purification apparatus according to Claim 7,
wherein one end of the fluid infusing line is connected to the dialysate introduction line, and the dialysate as the replenishment fluid is supplied to the arterial blood circuit or the venous blood circuit.

9. The priming method of the blood purification apparatus according to Claim 7 or 8,
wherein the fluid supplying device includes a fluid infusing pump disposed in the fluid infusing line.

10. The priming method of the blood purification apparatus according to any one of Claims 7 to 9,
wherein a proximal end of the fluid infusing line is connected to the arterial air trap chamber and a pre-fluid infusion is performed in the blood purification treatment process,
**characterized in that** during priming, the blood pump is driven in reverse rotation and a drive speed of the blood pump is set to be equal to or less than a supply speed of the replenishment fluid using the fluid supplying device.

11. The priming method of the blood purification apparatus according to any one of Claims 7 to 9,
wherein the proximal end of the fluid infusing line is connected to the venous air trap chamber and a post-fluid infusion is performed in the blood purification treatment process
**characterized in that** during priming, the blood pump is driven in normal rotation and a drive speed of the blood pump is set to be equal to or less than a supply speed of the replenishment fluid using the fluid supplying device.

12. The priming method of the blood purification apparatus according to Claim 11,
wherein an air bubble detection device is disposed at a tip side of the venous blood circuit, and during priming, the control device sequentially performs a first circulation process of driving the blood pump in reverse rotation at a predetermined speed and a second circulation process of driving the blood pump at a speed lower than the predetermined speed as a condition that the air bubble detection device detects air bubbles in the first circulation process.
